(19) Europäisches Patentamt
European Patent Office
Office européen des brevets

(11) Numéro de publication: **0 071 500**
**B1**

# (12) FASCICULE DE BREVET EUROPEEN

(45) Date de publication du fascicule du brevet: **22.05.85**

(51) Int. Cl.⁴: **C 07 C 102/00**, C 07 C 103/183, C 07 C 117/00

(21) Numéro de dépôt: **82401288.4**

(22) Date de dépôt: **08.07.82**

(54) **Procédé de préparation d'amino-4 butyramide.**

(30) Priorité: **29.07.81 FR 8114695**

(43) Date de publication de la demande: **09.02.83 Bulletin 83/6**

(45) Mention de la délivrance du brevet: **22.05.85 Bulletin 85/21**

(84) Etats contractants désignés: **AT BE CH DE FR GB IT LI LU NL SE**

(56) Documents cités:
**FR - A - 2 300 757**
**US - A - 3 880 922**

**E. MÜLLER "Methoden der organischen Chemie", vol.10/3, 1965, Georg Thieme Verlag, Stuttgart (DE), page 820**

(73) Titulaire: **SYNTHELABO, 58, rue de la Glacière, F-75621 Paris Cedex 13 (FR)**

(72) Inventeur: **Gosteli, Jacques, Cerecon A.G., CH-4416 Bubendof (CH)**

(74) Mandataire: **Thouret-Lemaitre, Elisabeth et al, Service Brevets - SYNTHELABO 58, rue de la Glacière, F-75621 Paris Cedex 13 (FR)**

**EP 0 071 500 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé pour la préparation d'amino-4 butyramide, de formule I,

$H_2NCH_2CH_2CH_2CONH_2$ (I)

et de ses sels. Ce dérivé de l'acide amino-4 butyrique présente un grand intérêt du fait de ses propriétés psychotropes (cf. V.M. Kopelevich, Usp. Khim. 48, 1273 (1979).

La synthèse connue composé (I), d'après laquelle on part du dinitrile de l'acide succinique, n'est pas adaptée aux exigences de la fabrication, en raison des mauvais rendements [C. Berther, Chem. Ber. 92, 2616 (1959)]. La méthode récemment publiée pour la préparation de (I) implique l'utilisation d'acide cyanhydrique libre, hautement toxique, et une hydrogénation sous pression, c'est-à-dire un ensemble de réaction exigeant des mesures de sécurité et des appareils particuliers [(A. Kleeman et coll., Angew. Chem. 92, 640 (1980)].

Conformément à l'invention, et de manière avantageuse, on prépare l'amino-4 butyramide (I) par réduction de l'azido-4 butyramide de formule II

$N_3CH_2CH_2CONH_2$ (II)

On connaît déjà des procédés de réduction d'azides en amides, pour des composés différents, par le brevet des Etats Unis d'Amerique N° 3 880 922 et par la demande de brevet français N° 2 300 757.

Selon l'invention la réduction est effectué par l'hydrogène en présence d'un catalyseur d'hydrogénation. On peut travailler sans appliquer de pression et à la température ambiante. Mais, on peut également utiliser d'autres agents réducteurs. Ainsi par exemple on peut d'abord faire réagir (II) avec une phosphine, telle que la triphénylphosphine, puis hydrolyser l'iminophosphine intermédiaire qui s'est formée et qui répond à la formule III

$R_3P = N\text{-}CH_2CH_2CH_2CONH_2$ (III)

(R = alkyle, phényle)

L'azido-4 butyramide, qui n'est pas décrit dans la littérature, peut être préparé de manière simple à partir de l'azido-4 butyronitrile connu, par hydrolyse partielle en milieu acide ou basique. Une méthode particulièrement avantageuse consiste à travailler en milieu basique, avec addition de peroxyde d'hydrogène.

Les exemples suivants illustrent l'invention.

*Exemple 1*

On hydrogène à pression et température ambiante, pendant 4 heures, une solution de 1,53 g (12 mM) d'azido-4 butyramide (II) dans 20 ml d'éthanol absolu et 3,22 ml d'acétate d'éthyle, contenant 12 mM de chlorure d'hydrogène, en présence de 1,53 g de palladium sur support de sulfate de baryum (5%).

Après filtration et évaporation du filtrat sous vide on recueille 1,13 g d'un résidu cristallin; par une extraction du catalyseur avec du méthanol chaud et évaporation sous vide on obtient une deuxieme fraction de 0,45 g du chlorhydrate brut, cristallisé, de l'amino-4 butyramide (I).

Rendement brut: 95%.

Moyennant une perte de produit, on peut recristalliser le produit brut dans l'alcool absolu. Le produit pur fond à 136-137°C.

*Exemple 2*

On agite à température ambiante 9,0 g (82 mM) d'azido-4 butyronitrile et 20 ml d'acide chlorhydrique concentré; après un certain temps il se forme une phase unique. Au bout de sept heures on concentre sous vide et on dilue les 19 g d'huile résiduelle avec 30 ml d'eau. En ajoutant avec précaution du carbonate de sodium solide, on ajuste le pH à 8, on extrait la solution quatre fois avec du chlorure de méthylène, et on sèche les extraits sur sulfate de sodium. Par une évaporation sous vide obtient 7,0 g de produit brut (II).

Rendement: 66%. Après recristallisation dans l'éther, l'azido-4 butyramide fond à 73-73,5°C.

$C_4H_8N_4O$.

| | | | |
|---|---|---|---|
| calculé: | C 37,49 | H 6,29 | N 43,73 |
| trouvé: | C 37,66 | H 6,33 | N 43,62 |

*Exemple 3*

A une solution de 23,3 g d'azido-4 butyronitrile dans 70 ml d'alcool on ajoute 46 ml d'une solution à 30% de peroxyde d'hydrogène. Tout en agitant on y introduit 17 ml de soude normale en veillant à ne pas dépasser une température de 37°C. Après cette addition on agite encore pendant deux heures, on chasse l'alcool par distillation dans un évaporateur à vide, on dilue le résidu avec un peu d'eau et on l'extrait deux fois avec du pentane. On jette l'extrait, on sature la phase aqueuse avec du chlorure de sodium et on l'agite six fois avec de l'acétate d'éthyle. On réunit les extraits et on les lave avec une solution de chlorure de sodium, on les sèche sur sulfate de sodium et on les concentre dans un évaporateur rotatif. On met le résidu cristallin en suspension dans de l'éther, on le porte brièvement à ébullition et, après refroidissement, on l'essore. Après séchage sous vide on recueille 17,7 g de cristaux (II) qui fondent à 73-73,5°C. Par concentration de la liqueur mère on obtient une seconde fraction de 5,3 g de cristaux (II) fondant à 70,5-71,5°C.

Rendement: 85%.

**Revendications**

1. Procédé de préparation d'amino-4 butyramide et de ses sels, caractérisé en ce que l'on réduit l'azido-4 butyramide par de l'hydrogène en présence d'un catalyseur d'hydrogénation.

2. Procédé selon la revendication 1, caractérisé en ce qu'on effectue la réduction à température ambiante et sous pression normale.

3. Procédé selon la revendication 1, caractérisé en ce qu'on prépare l'azido-4 butyramide par hydrolyse partielle de l'azido-4 butyronitrile.

4. Procédé selon la revendication 3, caractérisé en ce qu'on effectue l'hydrolyse en milieu aqueux en ajoutant du peroxyde d'hydrogène.

5. L'azido-4 butyramide, à titre de composé nécessaire comme intermédiaire dans un procédé selon l'une quelconque des revendications 1 à 4.

**Patentansprüche**

1. Verfahren zur Herstellung von 4-Aminobuttersäureamid und seinen Salzen, dadurch gekennzeichnet, dass 4-Azidobuttersäureamid mittels Wasserstoff in Gegenwart eines Hydrierungskatalysators reduziert wird.

2. Verfahren gemäss Anspruch 1, dadurch gekennzeichnet, dass die Reaktion bei Raumtemperatur und bei gewöhnlichem Druck vorgenommen wird.

3. Verfahren gemäss Anspruch 1, dadurch gekennzeichnet, dass das 4-Azidobuttersäureamid durch partielle Hydrolyse von 4-Azidobutyronitril hergestellt wird.

4. Verfahren gemäss Anspruch 3, dadurch gekennzeichnet, dass die Hydrolyse in wässrigem Milieu unter Zugabe von Wasserstoffperoxid vorgenommen wird.

5. 4-Azidobuttersäureamid als nötiges Zwischenprodukt in einem Verfahren gemäss irgendwelchem Anspruch 1 bis 4.

**Claims**

1. A process for the preparation of 4-aminobutyramide and salts thereof, characterized in that 4-azidobutyramide is reduced by hydrogen in the presence of a hydrogenation catalyst.

2. A process according to claim 1, characterized in that the reduction is carried out at ambient temperature and under normal pressure.

3. A process according to claim 1, characterized in that the 4-azidobutyramide is prepared by partial hydrolysis of 4-azidobutyronitrile.

4. A process according to claim 3, characterized in that the hydrolysis is carried out in an aqueous medium by adding hydrogen peroxyde.

5. 4-azidobutyramide, as a compound necessary as an intermediate in a process according to any of claims 1 to 4.